# EUROPEAN PATENT APPLICATION

(11) **EP 4 085 831 A1**
(43) Date of publication of application: **09.11.2022**
(21) Application number: 21172081.8
(22) Date of filing: 04.05.2021
(51) Int. Cl.: A61B 5/055, A61B 5/00, A61B 5/0205, A61B 5/11, A61B 6/04, A61G 7/00, A61B 5/16

(54) **MEDICAL IMAGING SYSTEM**

(71) Applicant: Koninklijke Philips N.V., 5656 AG Eindhoven (NL)
(72) Inventor: LEUSSLER, Christoph Günther, 5656 AE Eindhoven (NL); NORDHOFF, Tanja, 5656 AE Eindhoven (NL)
(74) Representative: Philips Intellectual Property & Standards

(57) **Abstract**

The present invention relates to medical imaging system (10), comprising:
- a movable bed (20);
- a medical imaging scanner (30);
- at least one sensor (40); and
- a processor unit (50).

The movable bed is configured to move a patient on the movable bed from a first area of a medical centre to a second area of the medical centre where the medical imaging scanner is located. The medical imaging scanner is intended to acquire at least one medical image of the patient. The at least one sensor is configured to acquire sensor information relating to the patient on the movable bed. The at least one sensor is configured to provide the sensor information to the processor unit. The medical imaging scanner is configured to provide scanner status information for the medical imaging scanner to the processor unit. The processor unit is configured to determine control information for the movable bed, the determination comprising utilization of the sensor information and the scanner status information.

## Description

### FIELD OF THE INVENTION

The present invention relates to a medical imaging system, a medical imaging method, as well as to a computer program element and a computer readable medium.

### BACKGROUND OF THE INVENTION

High patient throughput is crucial for many medical imaging facilities. At the same time, patient engagement and experience are becoming more important and are even part of the reimbursement in selected markets like the US. Moreover, imaging will become more and more autonomous in future with less operator depended actions and automated workflow steps. This leads to the need of good supporting tools to inform and guide operator and patient scenarios.

FlexTrak Mammo and the wide bore Ingenia MR system provide a comprehensive breast solution. Easy patient preparation, workflow and after-care is combined with high image quality, patient comfort and scanner efficiency. FlexTrak Mammo is available in combination with the dS Breast 16ch coil and/or dS Breast 7ch coil. An adjustable head rest with patient mirror and soft patient ramp are included to promote patient comfort.

Upcoming RF wireless coil technology applied in MRI signal transmission simplifies coil handling by the patient and operator for autonomous image acquisition. While wireless RF coil technology offers convenience and ease of operation, it introduces challenges in maintaining the phase information of individual signals, or powering the active parts of the RF coil. The combination of wireless coils, and detection of optimal relative overlap is a further challenge to be addressed. For autonomous preparation of a patient, different combinations of receive coil setups need to be selected as a function of region to be imaged or individually selected anatomy.

There is a need to resolve these issues.

### SUMMARY OF THE INVENTION

It would be advantageous to have an improved means of improving the throughput and patient comfort with respect to medical imaging. The object of the present invention is solved with the subject matter of the independent claims, wherein further embodiments are incorporated in the dependent claims. It should be noted that the following described aspects and examples of the invention apply to the medical imaging system, and also to the medical imaging method as well as to the computer program element and the computer readable medium.

In a first aspect, there is provided a medical imaging system, comprising:
- a movable bed;
- a medical imaging scanner;
- at least one sensor; and
- a processor unit.

The movable bed is configured to move a patient on the movable bed from a first area of a medical centre to a second area of the medical centre where the medical imaging scanner is located. The medical imaging scanner is intended to acquire at least one medical image of the patient. The at least one sensor is configured to acquire sensor information relating to the patient on the movable bed. The at least one sensor is configured to provide the sensor information to the processor unit. The medical imaging scanner is configured to provide scanner status information for the medical imaging scanner to the processor unit. The processor unit is configured to determine control information for the movable bed, the determination comprising utilization of the sensor information and the scanner status information.

Thus, patient workflow with respect to timely utilization of a medical image scanner is improved, because information relating to both the patient on a bed to be used to transport the patient to the scanner, derived from sensor data, and status information relating to the scanner is utilized, thereby enabling the patient to be delivered to the scanner at exactly the correct time for the scan.

In this manner, if the scanner is expected to be free for use earlier than expected, the patient can be expeditiously brought to the scanner, and conversely if a scan for a patient undergoing a scan is taking longer than necessary, the patient can be kept away from the scanner room until the scanner is ready.

Furthermore, by monitoring the patient, if the patient is not ready for a scan, for example sensor data shows that they have become agitated or stressed, they can be held back from being scanned, enabling another patient to "jump the queue" ahead of the agitated stressed patient, ensuring that the scanner's time is utilized to its maximum. Also, when the patient is determined to now be ready for the scan, they can re-join the queue and even jump to the front of the queue to minimise the amount of time they will have to wait for the scan.

It is to be noted that the second area of the medical centre can be for example comprises a mobile medical image scanner, such as a mobile MRI, installed on a truck. Thus, a patient can be moved from within a building to a truck. This also means that "medical centre" is here utilized in a general sense in terms of meaning an area where a patient is moved from one area or location where a medical image scanner is not located to an area or location where the medical image scanner is located.

In an example, the sensor information relating to the patient on the movable bed comprises vital signs data for the patient on the movable bed.

In an example, the vital signs data comprises one or more of: ECG data; anaesthesia data; heart beat data; blood pressure data; skin conductance data; breathing rate data; motion data of body parts determined from RF and/or optical sensing.

Thus, the physiological and/or emotional state of the patient can be taken into account.

In an example, the medical imaging scanner is an MRI image unit, an X-ray image unit, a PET image unit, a CT-Radiation therapy unit, or a MR-LINAC scanner.

In an example, the medical imaging scanner is an MRI image unit, and the at least one sensor comprises at least one wireless MRI compatible coil. The sensor information relating to the patient on the movable bed can then comprise status information relating to the at least one wireless MRI compatible coil.

In an example, the status information relating to the at least one wireless MRI compatible coil comprises one or more of: battery status of the wireless MRI compatible coil or coils; type of the coil or coils; the coil vendor or vendors; RF power limit of the coil or coils; operational temperature of the coil or coils; service data of the coil or coils; functionality data of the coil or coils.

In this manner, wireless MRI coils that are starting to be used with MRI scans can be set up beforehand, and positioned with respect to the patient during transit of the patient to the scanner and not delay scanning by undertaking this set-up at the scanner.

In an example, the control information for the movable bed comprises coil configuration information to configure the wireless MRI compatible coil with respect to the acquisition of the at least one medical image of the patient.

In an example, the processor unit is configured to determine configuration information for the medical imaging scanner. The determination comprises utilization of the sensor information and the scanner status information.

In an example, the scanner status information for the medical imaging scanner comprises an indication of whether the scanner is in use and/or an indication when the scanner will be available for use.

In this manner, the control information can take into account when the scanner is expected to be ready for the patient, enabling information/instructions/control to be provided to enable the bed with the patient to be brought to the scanner at exactly the correct time for the scan, and be kept out of the way until that time.

In an example, the system comprises one or more further movable beds. The scanner status information can then comprise sensor information relating to one or more other patients on the one or more further movable beds, and the medical imaging scanner is intended to acquire at least one medical image for each of the one or more other patients.

Thus, the system can take account of all the patients in their various stages of transit to the scanner, changing the patient scan order if necessary to allow agitated/stressed patients to calm down and prioritise cases such as emergencies or children, and plan the scans based on the readiness of equipment to be used in the scan etc.

In an example, the control information comprises instructions to control one or more brakes for one of more wheels of the movable bed.

In an example, the control information comprises instructions to control the provision of movement information to an operator. The movement information comprises information displayed on at least one visual display unit.

In an example, the control information comprises instructions to control the provision of movement information to an operator. The movement information comprises information output on at least acoustic output unit.

In an example, the at least one visual display unit comprises a visual display unit mounted to the movable bed.

In an example, the at least one visual display unit comprises a visual display unit separated from the movable bed.

In an example, the at least one acoustic output unit comprises an acoustic output unit mounted to the movable bed.

In an example, the at least one acoustic output unit comprises an acoustic output unit separated from the movable bed.

In a second aspect, there is provided a medical imaging method with a medical imaging system, the method comprising:
a) moving a patient on a movable bed from a first area of a medical centre to a second area of the medical centre where a medical imaging scanner is located, and wherein the medical imaging scanner is intended to acquire at least one medical image of the patient;
b) prior to moving the patient and/or during movement of the patient acquiring, by at least one sensor, sensor information relating to the patient on the movable bed;
c) providing the sensor information to a processor unit;
d) prior to moving the patient and/or during movement of the patient providing scanner status information for the medical imaging scanner to the processor unit; and
e) prior to moving the patient and/or during movement of the patient determining by the processor unit control information for the movable bed, the determining comprising utilizing the sensor information and the scanner status information.

According to another aspect, there is provided a computer program element controlling one or more of the systems as previously described which, if the computer program element is executed by a processing unit, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic set up of an example of a medical imaging system;
Fig. 2 shows a medical imaging method;
Fig. 3 shows an example of a patient bed with wireless access and integrated sensing;
Fig. 4 shows an example of patient preparation using a wireless link patient tray, where individual patient data (motion, breathing) is checked via wireless link;
Fig. 5 shows an example of communication of individual mobile patient bed with a state machine (or processor unit) via wireless link, where the individual data is used to control the MRI sequence, inform remote or local operator actions (MRI console), and connect to 3D sensing; and
Fig. 6 shows an example of a detailed workflow or algorithm of wireless link connection and control of workflow via feedback software loop.

### DETAILED DESCRIPTION OF EMBODIMENTS

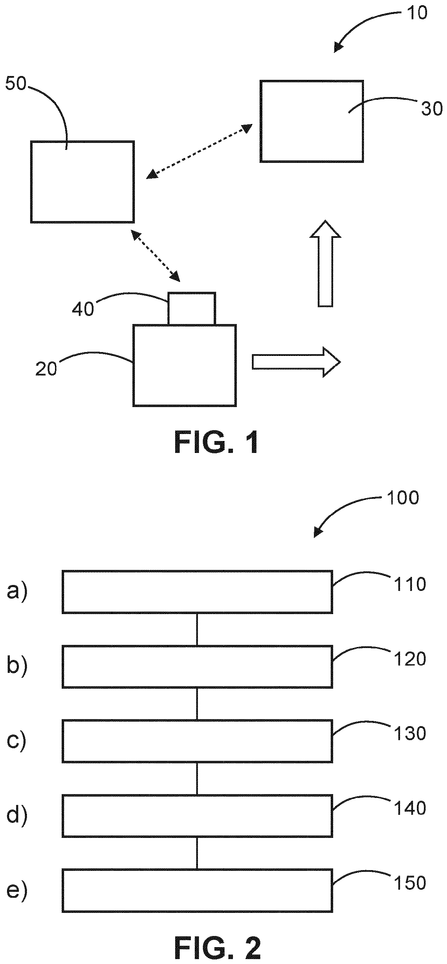

Fig. 1 shows a schematic example of a medical imaging system 10. The system comprises a movable bed 20, a medical imaging scanner 30, at least one sensor 40, and a processor unit 50. The movable bed is configured to move a patient on the movable bed from a first area of a medical centre to a second area of the medical centre where the medical imaging scanner is located. The medical imaging scanner is intended to acquire at least one medical image of the patient. The at least one sensor is configured to acquire sensor information relating to the patient on the movable bed. The at least one sensor is configured to provide the sensor information to the processor unit. The medical imaging scanner is configured to provide scanner status information for the medical imaging scanner to the processor unit. The processor unit is configured to determine control information for the movable bed, the determination comprising utilization of the sensor information and the scanner status information.

In an example, the processor unit is configured to provide the sensor information to the medical imaging scanner.

In an example, the processor unit is configured to provide the control information to the medical imaging scanner.

Thus, the medical imaging scanner, in terms of a controller or processing unit for the scanner, is also provided with a view of what is happening with respect to patients and their transit to the scanner.

In an example, the processor unit is configured to utilize the control information to provide a communication that an operator is required to come to the movable bed.

In an example, the processor unit is configured to utilize the control information to provide a communication that an operator is required to move the movable bed.

In an example, the processor unit is configured to utilize the control information to provide a communication that an operator is required to stop moving the movable bed.

In an example, the processor unit is contained with the movable bed.

In an example, the processor unit is separate to the movable bed.

In an example, the processor unit is configured to wirelessly communicate with the at least one sensor.

In an example, the processor unit is configured to wirelessly communicate with the medical imaging scanner.

In an example, the at least one sensor is connected to the movable bed.

In an example, the at least one sensor is separate to the movable bed.

In an example, the at least one sensor is configured to wirelessly communicate with the processor unit.

In an example, the at least one sensor is configured to wirelessly communicate with the medical imaging scanner.

For example, communication to the medical imaging scanner can be via intermediate repeater transponders distributed in the imaging centre. The medical imaging scanner can be connected via a digital network, cloud state machine with the transponders.

According to an example, the sensor information relating to the patient on the movable bed comprises vital signs data for the patient on the movable bed.

According to an example, the vital signs data comprises one or more of: ECG data; anaesthesia data; heart beat data; blood pressure data; skin conductance data; breathing rate data; motion data of body parts determined from RF and/or optical sensing.

In an example, the processor unit is configured to utilize the vital signs data to provide a communication that a medical expert and/or operator is required to come to the movable bed.

According to an example, the medical imaging scanner is an MRI image unit, an X-ray image unit, a PET image unit, a CT-Radiation therapy unit, or a MR-LINAC scanner.

According to an example, the medical imaging scanner is an MRI image unit, and the at least one sensor can comprise at least one wireless MRI compatible coil. The sensor information relating to the patient on the movable bed can then comprise status information relating to the at least one wireless MRI compatible coil.

According to an example, the status information relating to the at least one wireless MRI compatible coil comprises one or more of: battery status of the wireless MRI compatible coil or coils; type of the coil or coils; the coil vendor or vendors; RF power limit of the coil or coils; operational temperature of the coil or coils; service data of the coil or coils; functionality data of the coil or coils.

In an example, the at least one wireless MRI compatible coil comprises a plurality of wireless MRI compatible coils, and the status information comprises the number of coils on the patient.

In an example, the at least one wireless MRI compatible coil comprises a plurality of wireless MRI compatible coils, and the status information comprises their position on the patient.

In an example, the at least one wireless MRI compatible coil comprises a plurality of wireless MRI compatible coils, and the status information comprises their position relative to one another.

In an example, the at least one wireless MRI compatible coil comprises a plurality of wireless MRI compatible coils, and the status information comprises the number of coils on the patient, and their position on the patient and their position relative to one another.

Thus, individual coil elements can be deactivated if it is determined that the coil positioning is not optimal.

According to an example, the control information for the movable bed comprises coil configuration information to configure the wireless MRI compatible coil with respect to the acquisition of the at least one medical image of the patient.

In an example, the coil configuration information comprises one or more of: at least one switching time; digital filter characteristics; analogue filter characteristics; RF noise matching adaptation, integrated sensor activation characteristics; bandwidth; field strength; gradient strength, B0 static field, RF coil loop activation.

According to an example, the processor unit is configured to determine configuration information for the medical imaging scanner, the determination comprising utilization of the sensor information and the scanner status information.

In an example, the configuration information for the medical imaging scanner comprises a MRI scan sequence for the patient.

According to an example, the scanner status information for the medical imaging scanner comprises an indication of whether the scanner is in use and/or an indication when the scanner will be available for use.

According to an example, the system comprises one or more further movable beds, and the scanner status information comprises sensor information relating to one or more other patients on the one or more further movable beds, and the medical imaging scanner is intended to acquire at least one medical image for each of the one or more other patients.

According to an example, the control information comprises instructions to control one or more brakes for one of more wheels of the movable bed.

According to an example, the control information comprises instructions to control the provision of movement information to an operator, and the movement information comprises information displayed on at least one visual display unit. Additionally or alternatively the control information comprises instructions to control the provision of movement information to an operator, and the movement information comprises information output on at least acoustic output unit.

According to an example, the at least one visual display unit comprises a visual display unit mounted to the movable bed. Additionally or alternatively the at least one visual display unit comprises a visual display unit separated from the movable bed;

According to an example, the at least one acoustic output unit comprises an acoustic output unit mounted to the movable bed. Additionally or alternatively the at least one acoustic output unit comprises an acoustic output unit separated from the movable bed.

In an example, the control information controlling the brakes of the bed can be correlated with the control information providing visual and/or acoustic information to an operator, thus to inform about workflow status.

Thus, a display with a display mounted on the bed can indicate to an operator that the bed should now be moved, and this can be displayed visually to the operator and this can be accompanied by an acoustic signal or even synthesized voice to also inform the operator to move the bed, in case they are not looking at the display. At the same time, brakes on a wheel or wheels of the bed can be released enabling the bed to be moved. At an appropriate point the operator is informed to stop moving the bed, and the brakes applied to stop the bed from moving. The brakes can be applied in a gradual manner, rather than off/on enabling the operator to be informed due to the gradually increasing resistance that the bed should now be placed in a stationary orientation. Additionally or alternatively to the display and loudspeaker on the bed, the operator can have a portable unit, similar to a smartphone that informs them to move the bed and how and to where it should be moved.

Fig. 2 shows a medical imaging method 100 with a medical imaging system 10. The method is shown in its basic steps, and where step a) or at least parts of step a) can occur before or at the same time as a number of different steps as described below, and the method comprises:
in a moving step 110, also referred to as step a) moving a patient on a movable bed from a first area of a medical centre to a second area of the medical centre where a medical imaging scanner is located, and wherein the medical imaging scanner is intended to acquire at least one medical image of the patient;
   prior to moving the patient and/or during movement of the patient in an acquiring step 120, also referred to as step b), acquiring, by at least one sensor, sensor information relating to the patient on the movable bed;
in a providing step 130, also referred to as step c), providing the sensor information to a processor unit;
prior to moving the patient and/or during movement of the patient in a providing step 140, also referred to as step d), providing scanner status information for the medical imaging scanner to the processor unit; and
prior to moving the patient and/or during movement of the patient in a determining step 150, also referred to as step e), determining by the processor unit control information for the movable bed, the determining comprising utilizing the sensor information and the scanner status information.

In an example, the sensor information relating to the patient on the movable bed comprises vital signs data for the patient on the movable bed.

In an example, the vital signs data comprises one or more of: ECG data; anaesthesia data; heart beat data; blood pressure data; skin conductance data; breathing rate data; motion data of body parts determined from RF and/or optical sensing.

In an example, the medical imaging scanner is an MRI image unit, an X-ray image unit, a PET image unit, a CT-Radiation therapy unit, or a MR-LINAC scanner.

In an example, the medical imaging scanner is an MRI image unit, and the at least one sensor can comprises a wireless MRI compatible coil. The sensor information relating to the patient on the movable bed can comprise status information relating to the wireless MRI compatible coil.

In an example, the status information relating to the wireless MRI compatible coil comprises one or more of: battery status of the wireless MRI compatible coil; type of coil; coil vendor; RF power limit of the coil; operational temperature of the coil; service data of the coil; functionality data of the coil.

In an example, the control information for the movable bed comprises coil configuration information to configure the wireless MRI compatible coil with respect to the acquisition of the at least one medical image of the patient.

In an example, the coil configuration information comprises one or more of: at least one switching time; digital filter characteristics; analogue filter characteristics; RF noise matching adaptation, integrated sensor activation characteristics; bandwidth; field strength; gradient strength, B0 static field, RF coil loop activation.

In an example, prior to moving the patient and/or during movement of the patient the method comprises determining by the processor unit configuration information for the medical imaging scanner, the determining comprising utilizing the sensor information and the scanner status information.

In an example, the configuration information for the medical imaging scanner comprises a MRI scan sequence for the patient.

In an example, the scanner status information for the medical imaging scanner comprises an indication of whether the scanner is in use and/or an indication when the scanner will be available for use.

In an example, the scanner status information comprises sensor information relating to one or more other patients on one or more further movable beds, and wherein the medical imaging scanner is intended to acquire at least one medical image for each of the one or more other patients.

In an example, the control information comprises instructions to control one or more brakes for one of more wheels of the movable bed.

In an example, the control information comprises instructions to control the provision of movement information to an operator, wherein the movement information comprises information displayed on at least one visual display unit.

In an example, the at least one visual display unit comprises a visual display unit mounted to the movable bed; and/or wherein the at least one visual display unit comprises a visual display unit separated from the movable bed.

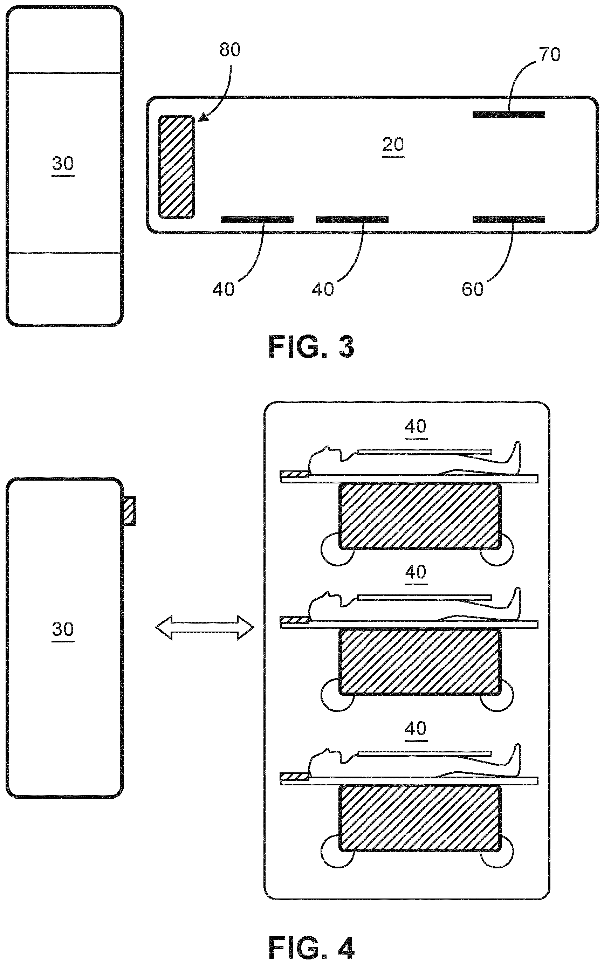

The medical imaging system and medical imaging method are explained in specific detail, where reference is made to Figs. 3-6. The specific embodiments centre on MRI imaging, but a system with other imaging modalities can also apply.

The inventors were aware of the following problems that existed with respect to the workflow aspects of coordinating patients for medical imaging in an efficient and timely manner, whilst maximizing the patient's comfort and experience.

Patient bed does have electrical connectors
RF coil check after coils are connected
B1 system power as a function of coil
When patient is prepared for example for mammo trak, no coil connection or sensing Functionality of coils is not checked
Several/Multiple patients cannot be prepared together
System needs optical/galvanic connection (connectors)
Wireless coils can be used on the patient and sensors can also be utilized, but functionality checks and placement indications have to be done when the patient is at the scanner causing a delay
No independent power source and no wireless link to remote operator
For multiple patients, each patient needs to be prepared and checked separately Monitoring of prepared patients
The new patient transfer bed and associated functionality within what can be termed a new overall medical imaging system was developed by the inventors to overcome these problems.

In summary, these problems were overcome through a new movable patient support or bed, which has an autonomous integrated wireless link allowing patient preparation and controlled workflow. Multiple patients can be prepared together, or at least a new patient can start to be prepared before an earlier patient has arrived at the scanner. RF coils and bed integrated sensors can checked remotely for vital signs data and to check if patient is ready for a scan. The dataflow is routed via a wireless link to a remote host (state machine or processor unit). To control the patient bed workflow, individual patient beds are equipped with electronically controlled brakes and display information to only allow the correct sequence of patients arriving at the scanner. Results of the individual measured data is fed to a state machine (processor unit), which configures the MR sequence and external control and projection elements. Communication of individual mobile patient bed with the state machine is via wireless link. The individual data is used to control, load individual MRI sequences, inform remote or local operator (MRI console) and connect/communicate to 3D sensing. An algorithm of wireless link connection and control of workflow is via feedback software loop.

Thus, wireless coils and sensing along with scanner status information enables the use of a mechanical patient bed/tray/support, which needs no connectors or cable routing. The patient bed has a wireless interface integrated and DC power battery, and information on the scanner and the patient is utilized to coordinate a patient's arrival at the scanner with respect to other patients. This allows for multiple guided patient preparation and a faster workflow

As shown in Figs. 3-4, a patient bed with a wireless link and integrated sensing is shown. The mobile patient bed is moved to the scanner and docking is directly performed without any galvanic or optical connection allowing faster and safe workflow. The MRI scanner is configured regarding individual coil and patient data. Multiple patients can be prepared simultaneously. Each patient has individual coils equipment, mattress and sensing. The individual state and workflow is automatically documented and checked with a patient data base.

In Fig. 3:
20 indicates patient bed wireless access,
30 indicates MRI scanner,
40 indicates sensor,
60 indicates switch,
70 indicates coil connect, and
80 indicates wireless link transceiver / battery.

In Fig. 4:
30 indicates MRI scanner,
40 indicates wireless coil, and

The imagery on the right represents the patients being prepared for scanning away from the scanner.

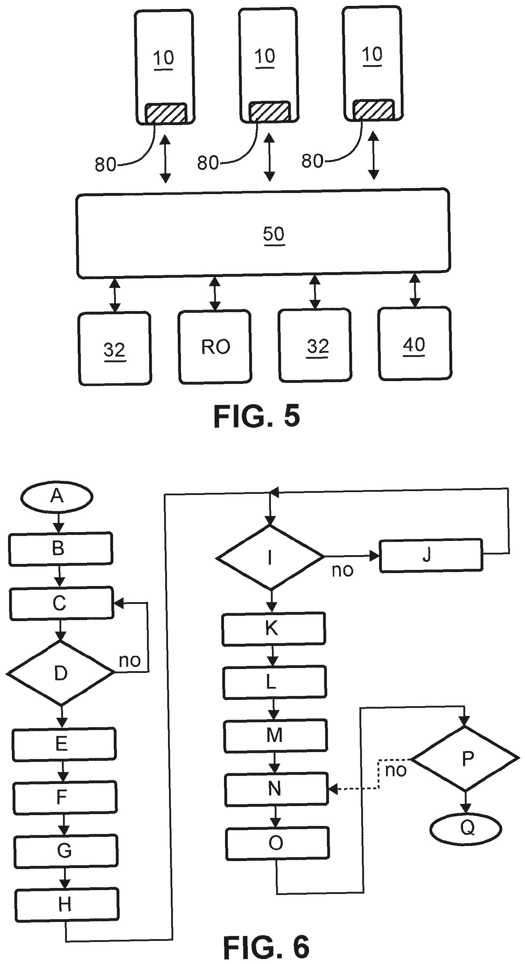

As shown in Figs. 5-6, the state machine (processor unit) directly processes the vital signs data and presents the information as textual or symbolic information. The data is continuously updated for the operator and MRI scanner. The operator obtains feedback information about motion or preceding events or user interactions and visualized one or more attribute parameter thresholds linked with a vital signs camera. The vital signs camera can be a 3D optical camera, 3D LIDAR or a 3D RF RADAR sensor. Detection of thresholds also come from the at least one sensor on the patient bed. If for example an anaesthesia unit is not ready, or going in service mode, then an appropriate action can to be taken by the computer program on the processing unit.

An algorithm of wireless link connection and control of workflow via feedback software loop runs on the state machine (processor unit).

In Fig. 5:
10 indicates bed 1, bed 2, and bed 3,
30 indicates MRI scanner,
32 indicates MRI console,
40 indicates 3D sensing,
50 indicates wireless link state machine (processor unit), and
RO indicates a remote operator.

In Fig, 6:
A indicates start
B indicates wireless link connect
C indicates prepare patient
D indicates ready
E indicates check coils
F indicates check vital signs
G indicates wireless link transmission
H indicates adapt and scan protocol
I indicates MRI ready
J indicates wait
K indicates transport
L indicates docking
M indicates load sequence
N indicates start scan
O indicates run protocols
P indicates MRI scan ready
Q indicates stop

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A medical imaging system (10), comprising:
- a movable bed (20);
- a medical imaging scanner (30);
- at least one sensor (40); and
- a processor unit (50);
wherein the movable bed is configured to move a patient on the movable bed from a first area of a medical centre to a second area of the medical centre where the medical imaging scanner is located;
wherein the medical imaging scanner is intended to acquire at least one medical image of the patient;
wherein the at least one sensor is configured to acquire sensor information relating to the patient on the movable bed;
wherein the at least one sensor is configured to provide the sensor information to the processor unit;
wherein the medical imaging scanner is configured to provide scanner status information for the medical imaging scanner to the processor unit; and
wherein the processor unit is configured to determine control information for the movable bed, the determination comprising utilization of the sensor information and the scanner status information.

2. System according to claim 1, wherein the sensor information relating to the patient on the movable bed comprises vital signs data for the patient on the movable bed.

3. System according to claim 2, wherein the vital signs data comprises one or more of: ECG data; anaesthesia data; heart beat data; blood pressure data; skin conductance data; breathing rate data; motion data of body parts determined from RF and/or optical sensing.

4. System according to any of claims 1-3, wherein the medical imaging scanner is an MRI image unit, an X-ray image unit, a PET image unit, a CT-Radiation therapy unit, or a MR-LINAC scanner.

5. System according to claim 4, wherein the medical imaging scanner is an MRI image unit, and wherein the at least one sensor comprises at least one wireless MRI compatible coil, and wherein the sensor information relating to the patient on the movable bed comprises status information relating to the at least one wireless MRI compatible coil.

6. System according to claim 5, wherein the status information relating to the at least one wireless MRI compatible coil comprises one or more of: battery status of the wireless MRI compatible coil or coils; type of the coil or coils; the coil vendor or vendors; RF power limit of the coil or coils; operational temperature of the coil or coils; service data of the coil or coils; functionality data of the coil or coils.

7. System according to any of claims 5-6, wherein the control information for the movable bed comprises coil configuration information to configure the wireless MRI compatible coil with respect to the acquisition of the at least one medical image of the patient.

8. System according to any of claims 1-7, wherein the processor unit is configured to determine configuration information for the medical imaging scanner, the determination comprising utilization of the sensor information and the scanner status information.

9. System according to any of claims 1-8, wherein the scanner status information for the medical imaging scanner comprises an indication of whether the scanner is in use and/or an indication when the scanner will be available for use.

10. System according to any of claims 1-9, wherein the system comprises one or more further movable beds, and wherein the scanner status information comprises sensor information relating to one or more other patients on the one or more further movable beds, and wherein the medical imaging scanner is intended to acquire at least one medical image for each of the one or more other patients.

11. System according to any of claims 1-10, wherein the control information comprises instructions to control one or more brakes for one of more wheels of the movable bed.

12. System according to any of claims 1-11, wherein the control information comprises instructions to control the provision of movement information to an operator, wherein the movement information comprises information displayed on at least one visual display unit; and/or wherein the movement information comprises information output on at least acoustic output unit.

13. System according to claim 12, wherein the at least one visual display unit comprises a visual display unit mounted to the movable bed; and/or wherein the at least one visual display unit comprises a visual display unit separated from the movable bed; and/or wherein the at least one acoustic output unit comprises an acoustic output unit mounted to the movable bed; and/or wherein the at least one acoustic output unit comprises an acoustic output unit separated from the movable bed.

14. A medical imaging method (100) with a medical imaging system (10), the method comprising:
a) moving (110) a patient on a movable bed from a first area of a medical centre to a second area of the medical centre where a medical imaging scanner is located, and wherein the medical imaging scanner is intended to acquire at least one medical image of the patient;
b) prior to moving the patient and/or during movement of the patient acquiring (120) by at least one sensor sensor information relating to the patient on the movable bed;
c) providing (130) the sensor information to a processor unit;
d) prior to moving the patient and/or during movement of the patient providing (140) scanner status information for the medical imaging scanner to the processor unit; and
e) prior to moving the patient and/or during movement of the patient determining (150) by the processor unit control information for the movable bed, the determining comprising utilizing the sensor information and the scanner status information.

15. A computer program element for controlling a system according to any one of claims 1 to 13, which when executed by a processor is configured to carry out the method of claim 14.
